# EUROPÄISCHE PATENTANMELDUNG

(11) **EP 0 955 544 A1**
(43) Veröffentlichungstag der Anmeldung: **10.11.1999**
(21) Anmeldenummer: 98108220.9
(22) Anmeldetag: 06.05.1998
(51) Int. Cl.: G01N 33/53

(54) **Verfahren zum Erfassen von Primärsignalen bei der Zellkommunikation von Zellen des Immunsystems**

(71) Anmelder: Hölzel Diagnostika Handels GmbH, 50670 Köln (DE)
(72) Erfinder: Hölzel, Veit, 50733 Köln (DE)
(74) Vertreter: Patentanwälte Sternagel & Fleischer

(57) **Zusammenfassung**

Die Erfindung betrifft ein Verfahren zum Erfassen von Primärsignalen der Zellkommunikation von menschlichen und Säugetierzellen außerhalb eines Organismus, wobei Polymerkügelchen mit einem Durchmesser von 5 bis 100 µm. die mindestens mit einem für diese Primärsignale spezifischen Antikörper beladen sind, mit menschlichen oder Säugetierzellen, die Primärsignale aussenden können, gemischt und in Zellkultur unter physiologischen Bedingungen inkubiert werden, worin die Zellen durch einen externen Reiz zur Abgabe des Primärsignals stimuliert werden und die Polymerkügelchen die abgegebenen Primärsignale auffangen, welche anschließend mit Hilfe eines weiteren Antikörpers, an den ein die Detektion zulassendes Molekül gekoppelt ist, meßbar gemacht werden.

## Beschreibung

Die vorliegende Erfindung betrifft ein außerhalb eines Organismus durchführbares Verfahren zur qualitativen und quantitativen Erfassung von Signalen, die bei der Kommunikation von Zellen des menschlichen oder tierischen Immunsystems ausgesendet werden.

Eukaryotische Zellen sind auf vielerlei Arten in der Lage anderen Zellen Informationen zu übermitteln. Dafür können sie, müssen aber nicht notwendigerweise miteinander in Kontakt treten. Eine Möglichkeit beispielsweise ist die Abgabe bestimmter Substanzen durch eine Zelle, die von einer anderen Zelle als Signal erkannt werden können. Eine bekannte Gruppe von solchen in der Zellkommunikation verwendeten Molekülen ist die der sogenannten "Cytokine". Sie werden unter anderem bei der Kommunikation der Zellen des Immunsystems verwendet. Zur Familie der Cytokine gehören alle bekannten Interleukine, wie auch die Interferone (IFN) α, β und γ, Tumor-Nekrose-Faktoren (TNF) und viele weitere Gruppen. Die besondere Eigenschaft dieser Stoffe besteht darin, daß sie auch in geringen Dosen sehr stark wirksam sind und daß ihre Wirkung die Zielzellen stark modifiziert. So können Cytokine in der Zielzelle z.B. deren Proliferation bewirken, die Proteinexpression beeinflussen und die Zelldifferenzierung steuern. Weil die Cytokine diese enormen Auswirkungen auf ihre Zielzellen haben, müssen sie extrem streng reguliert werden. Das Immunsystem reagiert auf einen Fremdkörper oder einen anderen auslösenden Faktor in den meisten Fällen mit einer lokal sehr eng begrenzten Antwort, wobei nur die Zellen involviert sind, die sich in nächster Nähe des auslösenden Faktors befinden. Auch scheinen die Zellen, die den Fremdkörper erkannt haben, vor allem dann Cytokine abzugeben, wenn sich Zielzellen in deren unmittelbarer Nähe befinden, die dann nach ihrer Aktivierung den Fremdkörper angreifen und beseitigen. Um auch nur diese Zellen in "Alarmbereitschaft" zu versetzen und dadurch die Immunantwort auszulösen, werden die Cytokine sehr schnell nach ihrer Abgabe in das Blut daraus wieder entfernt. Dies geschieht entweder durch sehr schnellen Abbau, durch Wiederaufnahme der Cytokine (entweder in die Ursprungszelle oder in die Zielzelle) oder durch "Abfangen" der Cytokine durch bluteigene Stoffe. Diese extreme zeitliche sowie lokale Begrenzung der Cytokine auf die unmittelbare Nachbarschaft der sie produzierenden Zellen hat zur Folge, daß sich die in der Immunantwort verwendeten Primärsignale nur sehr schwer erfassen und quantitativ messen lassen.

Eine allgemein bekannte Methode zur Erkennung von Molekülen ist die sogenannte ELISA-Methode (Enzyme-Linked Immuno-Sorbent Assay). Hierbei wird zunächst ein spezifischer primärer Antikörper gegen das zu detektierende Molekül ("Antigen") eingesetzt, der dieses Molekül hochspezifisch erkennt und bindet. Diese Bindung ist allerdings diffusionskontrolliert und somit konzentrationsabhängig. Der entstehende Antigen-Antikörper-Komlex muß nach der Bindung immobil sein, d.h. entweder wird ein freies Antigen durch einen verankerten Antikörper gebunden oder ein an eine Oberfläche gekoppeltes Antigen durch einen freien Antikörper erkannt. Nach einer gewissen Inkubationszeit wird ein zweiter Antikörper zugegeben, der spezifisch entweder gegen den Komplex erster Antikörper-Antigen oder gegen einen anderen Bereich des Antigens (der nach der Bindung an den primären Antikörper noch zugänglich ist) gerichtet ist. An diesen zweiten Antikörper ist ein Enzym gekoppelt, das eine Reaktion katalysiert, die eine Analyse der entstandenen Komplexe erlaubt. Der zweite Antikörper wird in so hohem Überschuß zugesetzt, daß alle primären Antikörper-Antigen-Komplexe erkannt werden. Nach einem Waschschritt, der überschussigen zweiten Antikörper entfernt, wird dem Ansatz ein Substrat zugegeben, das durch das an den zweiten Antikörper gekoppelte Enzym zu einem leicht meßbaren Produkt umgesetzt wird, meistens einem Farbstoff.

Eine andere Methode der angewandten Zellbiologie ist das Markieren bestimmter Oberflächenmoleküle von Zellen, z.B. um deren Vorkommen auf bestimmten Zellen nachzuweisen, Zellen bestimmter Eigenschaften voneinander zu trennen, einzelne Zellen zu betrachten, die Verteilung bestimmter Merkmale zu untersuchen oder ähnliches. Für diese Markierung von Zellen sind kleine Kügelchen bekannt, die aus Polymeren wie z.B. Polystyrol oder ähnlichen inerten Materialien hergestellt und mit Antikörpern beladen sind, die spezifisch gegen das gewünschte Oberflächenmolekül gerichtet sind. Diese Kügelchen, in der Literatur als "Beads" bekannt, sind üblicherweise darüberhinaus mit einem Farbstoff beladen, so daß sie nach der Bindung an das Oberflächenmolekül als Farbpunkte auf der Zelloberfläche der Zielzelle zu erkennen sind. Meistens handelt es sich hierbei um einen fluoreszierenden Farbstoff, der schnell und leicht identifiziert werden kann. So markierte Zellen können mittels einer bekannten Methode, der sogenannten Flow Cytometry leicht quantitativ detektiert werden.

Es besteht auch die Möglichkeit, die Beads magnetisch zu beladen. Zellen, die mit solchen magnetischen Beads markiert sind, können sehr leicht von anderen Zellpopulationen abgetrennt werden, indem sie in ein magnetisches Feld gebracht werden. Diese Methode, bekannt als MACS (Magnetic activated Cell Sorting), ist mittlerweile eine der gängigsten Methoden zum Abtrennen von Zellen, von denen das Muster der Oberflächenmoleküle bekannt ist.

Beads, die für solche Markierungen verwendet werden, sind deutlich kleiner als die damit markierten Zellen, damit sie fest an der Zelloberfläche haften bleiben. Der übliche Durchmesser bei dieser Verwendung liegt im Bereich von 50 nm.

Es ist verständlich, daß sich die Methodik des ELISA auch anwenden läßt, indem man den immobilen Anteil - nämlich das Antigen bzw. den primären Antikörper - anstatt an eine Plastikoberfläche auch an solche inerten Polymerkügelchen binden kann, die sich durch Zentrifugation aus dem Inkubationsansatz entfernen lassen. Bei dieser Anwendung von Beads werden - entgegen dem zuvor Beschriebenen - sogenannte "Macrobeads" verwendet, die wesentlich größer als eukaryotische Zellen sind, nämlich mit Durchmessern im mm-Bereich. Diese lassen sich nach dem ersten Inkubationsschritt leicht auch von Zellen wieder trennen. In diesem Fall ersetzt also das Bead die Plastikoberfläche des Reaktionsgefäßes.

Das Prinzip des ELISA - in der Sonderform des ELISpot - wird in der Veröffentlichung von J. Rönnelid & L. Klareskog (J. Immunol. Meth. *200* (1997); 17-26) für die Untersuchung der Produktion von Interferon-γ (IFN-γ) in humanen Immunzellen (sogenannten PBMC, Peripheral Blood Mononuclear Cells) beschrieben. Hier werden Anti-IFN-γ-Antikörper an die Böden von Plastik-ELISA-Platten gebunden und darauf eine Suspension von Zellen gegeben, die zuvor mit Hilfe von Mitogenen, die die Fremdkörperwirkung nachahmen, zur Cytokinabgabe stimuliert wurden. Nach einer gewissen Inkubationszeit werden die Zellen wieder entfernt und der zweite Antikörper zugegeben. Die Analyse erfolgt hier mit Hilfe der Avidin-Alkalischen Phosphatase, die einen Farbstoff erzeugt. Bei diesem Ansatz wird durch Auszählen der einzelnen Farbspots unter dem Mikroskop ermittelt, wieviel Prozent der Zellen IFN-γ bilden. Dabei gibt die Größe der Farbspots die relative Stärke der IFN-γ-Bildung wieder.

In einer Veröffentlichung von T. M. McHugh *et al*. in J. Immunol. Meth. *116* (1989); 213 - 219 wird die Anwendung von Beads verschiedener Größe zur Diskriminierung unterschiedlicher Antikörper in humanem Serum beschrieben. Hierbei werden Beads mit drei verschiedenen Durchmessern im µm-Bereich verwendet. Jeweils Beads einer bestimmten Größe werden mit einer bestimmten Präparation eines humaninfektlösen Pilzes (Candida albicans) beladen. Durch die verschiedene Präparation entsteht jeweils ein spezifisches Antigen-Gemisch, so daß später in Korrelation zu der Beadgröße unterschieden werden kann, ob im menschlichen Serum Antikörper gegen Zellwandbestandteile der Pilzzellen, gegen cytosolische Bestandteile oder gegen Gesamtzellextrakt vorhanden sind. Mit diesem Ansatz ist eine frühere oder akute Pilzinfektion nachweisbar, da ausschließlich das seit der Infektionsabwehr bestehende Vorhandensein von Antikörpern gegen einen oder mehrere Bestandteile der Pilzzellen im menschlichen Serum nachgewiesen wird. Hier ist ein Abtrennen der Beads aus dem Reaktionsansatz nicht nötig, da sich im Serum keine Blutzellen befinden.

A.C. Akdis *et al*. beschreiben in ihrem Artikel in 3. Immun. Meth. *182* (1995); 251 - 261 ein Testverfahren, bei dem Macrobeads mit einem Durchmesser von 6,35 mm, beladen mit Anti-IFN-γ-Antikörpern für die Detektion von IFN-γ eingesetzt werden. Der Nachweis erfolgt mit einem zweiten, freien Antikörper, der ebenfalls gegen IFN-γ gerichtet ist, jedoch gegen den nicht vom ersten Antikörper gebundenen Bereich. Der zweite Antikörper ist mit einem Fluoreszenzfarbstoff markiert, der eine Auswertung des Tests in einem Flow Cytometer zuläßt. Es sind drei Ansatzmöglichkeiten für den Test beschrieben: 1. Die Macrobeads werden direkt einer Zellkultur von Immunzellen (PBMC oder T-Lymphozyten) zugegeben, die Zellen durch Mitogene zur IFN-γ-Abgabe stimuliert, und anschließend wird freier (zweiter) Antikörper zugegeben, 2. es wird zunächst mit dem freien Antikörper das durch Stimulation abgegebene Signal gebunden und anschließend Macrobeads mit dem gebundenen Antikörper zugegeben, 3. beide Antikörper - gebundener und freier - befinden sich von Anfang an in der Zellkultur. Bei diesem Testverfahren gemäß dem ELISA dienen die Macrobeads lediglich dem Ersatz der Plastikoberfläche zur Bindung des Antikörpers. Es wird beschrieben, daß sie keinerlei Einfluß auf die Zellen nehmen, weder die Stimulierung noch die produzierte Menge des IFN-γ verändern und auch keine Zellproliferation bewirken. Auch der freie Antikörper hat keinen Einfluß auf die von der Zelle freigesetzte IFN-γ-Menge oder die Proliferation. Wie zuvor beschrieben wird ein Cytokin sehr schnell von der es abgebenden Zelle oder der Zielzelle wieder aufgenommen, oder durch Abbau zerstört. Ist das IFN-γ an einen der beiden Antikörper gebunden, ist es vor diesen Einflüssen bewahrt. Das sogenannte "Trapping", nämlich das Einfangen des von der Zelle freigesetzten IFN-γ, bevor es wieder aus dem Medium entfernt wird, hilft somit die Bestimmung der freigesetzten Menge im Zellkultur-Medium zu verbessern. In dieser Veröffentlichung wird in einer PBMC-Kultur, also einer Mischkultur verschiedener Zellen des Immunsystems ein maximaler Trapping-Faktor von 24,7 erreicht, d.h. es kann ca. die 20-fache Menge an IFN-γ nach der Stimulation durch ein Mitogen nachgewiesen werden verglichen zu einer Kultur, bei der stimuliert, aber nicht "getrappt" wird. In einer Kultur von gereinigten T-Lymphozyten, also nach Entfernung der Zielzellen für das abgegebene Cytokin verringert sich dieser Faktor auf 3,3. Dies zeigt, daß nicht allein die mitogene Stimulierung die abgegebene Menge an Cytokin bestimmt, sondern vermutlich auch die Wechselwirkung zwischen einer stimulierten Zelle und einer Zielzelle.

Aufgabe der vorliegenden Erfindung ist es, Primärsignale der Zellkommunikation, vor allem die Cytokin-Freisetzung von Zellen des Immunsystems außerhalb eines Gesamtorganismus zu erfassen, und sie stabil qualitativ und quantitativ meßbar zu machen.

Diese Aufgabe wird gelöst durch ein Verfahren zum Erfassen von Primärsignalen der Zellkommunikation von menschlichen und Säugetierzellen außerhalb eines Organismus, wobei Polymerkügelchen mit einem Durchmesser von 5 bis 100 µm, die mindestens mit einem für diese Primärsignale spezifischen Antikörper beladen sind, mit menschlichen oder Säugetierzellen, die Primärsignale aussenden können, gemischt und in Zellkultur unter physiologischen Bedingungen inkubiert werden, worin die Zellen durch einen externen Reiz zur Abgabe des Primärsignals stimuliert werden und die Polymerkügelchen die abgegebenen Primärsignale auffangen, welche anschließend mit Hilfe eines weiteren Antikörpers, an den ein die Detektion zulassendes Molekül gekoppelt ist, meßbar gemacht werden.

Die in dem Verfahren verwendeten Polymerkügelchen werden im Folgenden als "Beads" bezeichnet, wobei die Verwendung von "Beads" und "Polymerkügelchen" als Synonym zu verstehen ist. Die von den Zellen abgegebenen und mit Hilfe des erfindungsgemäßen Verfahren erfaßten Primärsignale sind vor allem Cytokine, auf die weiter unten näher eingegangen wird.

Bei dem erfindungsgemäßen Verfahren werden bevorzugt Zellen des Immunsystems mit Polymerkügelchen der Größe 5 bis 100 µm, besser 5 bis 30 µm, bevorzugt 6 bis 10 µm, im Idealfall - für die Adaption am Flowcytometer - 7 µm, die mit Antikörpern gegen ein oder mehrere Cytokin(e) beladen sind in unmittelbare Nähe gebracht, so daß ein direkter Kontakt zwischen den Kügelchen und den Zellen erfolgen kann. Die von der Zelle abgegebenen Signale werden somit von den auf den Kügelchen verankerten spezifischen Antikörpern in unmittelbarer Nähe der Zelle aufgefangen, und anschließend mit Hilfe eines zweiten Antikörpers, an den ein die Detektion der Cytokin-Antikörper-Komplexe erlaubendes Molekül gebunden ist, quantitativ ausgewertet, wobei mit größer werdendem Beaddurchmesser die Qualität des Primärsignal zu messen geringer wird, d.h. die Konzentrationen, die gemessen werden können, kleiner werden.

Bevorzugte Ausführungsformen sind den Unteransprüchen zu entnehmen.

Bei der hier beschriebenen Erfindung wird ein Verfahren vorgestellt, mit dessen Hilfe sich außerhalb eines Organismus Primärsignale von Zellen des Immunsystems erfassen lassen und quantitativ ausgewertet werden können.

Das Immunsystem von Menschen und Säugetieren ist in ständiger Bereitschaft, um körperfremde Stoffe sofort beim Auffinden zu eliminieren. Vor allem die weißen Blutkörperchen (Leukozyten) spielen eine äußerst wichtige Rolle bei der Immunantwort. Ununterbrochen findet eine Kontrolle aller Partikel statt, auf die die patrouillierenden weißen Blutkörperchen treffen. Hierfür ist auch die Antigen-Präsentation wichtig, die vor allem durch aktivierte Makrophagen, B-Lymphozyten aber auch durch bestimmte Gewebezellen erfolgt. Wird ein Antigen präsentiert, das als körperfremd erkannt wird, tritt eine sofortige Immunantwort ein. Diese Wechselwirkung zwischen den einzelnen Zellen des Immunsystems bedarf einer sehr genau abgestimmten Kommunikation, um eine falsche Immunantwort zu vermeiden.

Die an der Immunantwort beteiligte Zellen sind, geordnet nach Zelltyp, Zellart und Hauptfunktion:
1. weiße Blutkörperchen (Leukozyten)
   1.1. Granulozyten
      a) Neutrophile: phagocytosieren und zerstören Bakterien
      b) Eosinophole: zerstören größere Parasiten und modulieren allergische Entzündungsreaktionen
      c) Basophile: setzen bei Immunantworten Histamin und Serotonin frei
   1.2. Monozyten
      a) Makrophagen: phagocytosieren Mikroorganismen, Fremdkörper und alte Zelle
   1.3. Lymphozyten
      a) B-Zellen: produzieren und sezernieren Antikörper
      b) T-Zellen (T-Helfer-Zellen, Supressor-T-Zellen, Killer-T-Zellen): töten Virus-infizierte Zellen und regulieren die Aktivität anderer Leukozyten
   1.4. natürliche Killerzellen: töten Virus-infizierte Zellen und einige Tumorzellen
2. Gewebezellen
   2.1. dentritische Zellen (lymphoide Organe): Antigen-Präsentation
   2.2. Langerhans'sche Zellen (Haut): Antigen-Präsentation
   2.3. Thymus-Epithelzellen: Antigen-Präsentation
   2.4. Makrophagen (Bindegewebe): Antigen-Präsentation und Phagocytose von Fremdkörpern

Vor allem sind es die T-Lymphozyten, die regulierend in die Immunantwort eingreifen. T-Lymphozyten können dabei eine Zelle zur Zerstörung der Fremdkörper aktivieren - dies erfolgt vor allem durch die T-Helfer-Zellen - aber auch eine überschießende Immunantwort hemmen (Supressor-T-Zellen). Die Immunantwort ist eine extrem fein abgestimmte Reaktion der beteiligten Zellen aufeinander. Um diese feine Abstimmung zu erreichen, bedarf es einer schnellen, hochspezifischen und effektiven Kommunikation, d.h. dem Aussenden und Empfangen wirksamer und genau regulierbarer Signale. Die für diese Kommunikation verwendeten Cytokine entsprechen genau den für eine solche Feinabstimmung benötigten Signalen: sie sind sehr schnell sezernierbar, da sie in den Zellen fertig synthetisiert gespeichert werden, hochspezifisch und leicht und schnell wieder aus dem Blut zu entfernen. Beispiele für solche Cytokine sind wie folgenden Tabelle 1 angegeben, wobei dies keine erschöpfende Auflistung darstellt.

**Tabelle 1**

| Cytokine, Beispiele | | | |
|---|---|---|---|
| Cytokin | zellulärer Ursprung | Zielzelle | Wirkung |
| IL-1 | Antigen-präsentierende Zellen | T-Helfer-Zellen, | T-Zell- und B-Zell-Aktivierung |
| | | B-Zellen | |
| IL-2 | bestimmte T-Helfer-Zellen | aktivierte | stimuliert Proliferation |
| | | T-Zellen | |
| IL-3 | bestimmte T-Helfer-Zellen, Mastzellen | best. hämatopoetische Zellen | stimuliert Proliferation und Differenzierung |
| IL-4 | bestimmte T-Helfer-Zellen | B-Zellen, | stimuliert Proliferation, Erhöhung der Anitgen-Präsentation auf B-Zellen |
| | | T-Zellen, Mastzellen | |
| IL-5 | bestimmte T-Helfer-Zellen | B-Zellen, Eosinophile | Erhöhung der Proliferation, Differenzierung |
| IL-6 | bestimmte T-Helfer-Zellen, Monozyten, Makrophagen | aktivierte | T-Zell-Aktivierung, Differenzierung von B-Zellen zu Plasmazellen |
| | | B-Zellen, | |
| | | T-Zellen | |
| IL-7 | Stroma-Zellen des Knochenmarks und Thymus | bestimmte Prä-B-Zellen | stimuliert Proliferation, Differenzierung von B-Zellen |
| IL-8 | Endothelzellen | Neutrophile | Neutrophilen-Aktivierung zur Chemotaxis |
| IL-9 | bestimmte T-Helfer-Zellen | T-Zellen | Unterstützt die Proliferation |
| IL-10 | T-Helfer-Zellen | Makrophagen | Blockierung der Produktion und Freisetzung anderer Cytokine |
| IL-11 | Stroma-Zellen des Knochenmarks | Plasmazellen, Prä-B-Zellen | Differenzierung |
| IL-12 | Makrophagen, B-Zellen, dendritische Zellen | aktivierte T-Zellen | Differenzierung von Lymphozyten zu T-Helfer-Zellen |
| IL-13 | bestimmte T-Helfer-Zellen | Makrophagen | Inhibierung der Freisetzung inflammatorischer Cytokine |
| IL-15 | Endothelzellen, Epithelzellen | T-Zellen | T-Zell-Aktivierung |
| IL-16 | CD8⁺ T-Zellen | Monozyten, Eosinophile | Anregung von Eosinophilen und Monozyten zur Chemotaxis |
| IL-17 | | | |
| IL-18 | | | |
| IFN-γ | bestimmte T-Helfer-Zellen | Makrophagen, B-Zellen, T-Zellen, diverse | unterstützt verschiedene Zellreaktionen bei der Immunantwort |
| TNF-α | Makrophagen | Tumor-Zellen, Entzündungszellen | verhindert Zellwachstum, cytotoxischer Effekt, |
| TNF-ß | T-Zellen | Tumor-Zellen, Makrophagen, Neutrophile | Cytotoxischer Effekt auf Tumorzellen, verstärkt Phagocytose |
| TGF-ß | Macrophagen, Lymphozyten | Monozyten, Makrophagen | Induziert und verstärkt die IL-1 Produktion |
| Abkürzungen: IL = Interleukin, IFN = Interferon, TNF = Tumor-Nekrose-Faktor, TGF = Transforming growth factor | | | |

Bevorzugt soll mit dem erfindungsgemäßen Testverfahren ein Primärsignal in Form eines Cytokins untersucht werden, das von einer Zelle ausgesendet wird, die an der Immunantwort beteiligt ist.

Für das Einfangen der Primärsignale werden Polymerkügelchen verwendet, die mit einem spezifischen Antikörper gegen das zu detektierende Cytokin beladen sind. Es wurde überraschend festgestellt, daß mit Polymerkügelchen mit einem Durchmesser von 5 - 100 µm ein sehr viel stärkeres Signal und somit eine sehr viel höhere Signalqualität erhalten werden konnte, als aus bisherigen Untersuchungen zu erwarten gewesen war.

Diese Größe der Polymerkügelchen ist vermutlich deswegen geeignet, da sie in etwa der Größe von Zellen des Immunsystems entspricht, und Beads dieser Größe mit den Zellen in direkten Kontakt treten können und dabei die größtmögliche Oberfläche bieten. Zellen des Immunsystems sind, wie schon zuvor beschrieben, bei der Kommunikation auf unmittelbare Nähe, bzw. auf direkten Kontakt angewiesen. Polymerkügelchen der erfindungsgemäß bevorzugten Größe können mit den Zellen in diesen direkten Kontakt treten und scheinen eine Zielzelle des Immunsystems nachzuahmen, so daß die Zelle, deren Primärsignal untersucht werden soll zur Abgabe dieses Signals in voller Stärke angeregt wird. Kleinere Kügelchen (Durchmesser von etwa 0,5 - 3 µm) können phagocytosierende Zellen des Immunsystems zur Phagocytose anregen, d.h. sie werden als Fremdkörper betrachtet. Noch kleinere Kügelchen, wie sie beispielsweise für die Markierung der Zelloberfläche verwendet werden, können die Zell-Zell-Wechselwirkung nicht mehr nachahmen. Auch bei der Verwendung größerer Beads werden diese nicht als "Nachbarzelle" angesehen, sondern - wie z.B. in herkömmlichen ELISA-Tests gezeigt - wie eine Oberfläche betrachtet. Aus diesen Gründen ist die bevorzugte Größe der in dem erfindungsgemäßen Testverfahren verwendeten Polymerkügelchen im Größenordnungsbereich einer Blutzelle des Immunsystems.

Das verwendete Polymer zur Herstellung der Beads muß aus einer Gruppe von inerten, biokompatiblen Substanzen ausgewählt werden. Beispiele hierfür sind Homo- oder Copolymere aus Styrol, Methylstyrol, Acrylamid/N,N,-Bisacrylamid, 2-Hydroxyethyl-Methacrylat, Methacrylsäure und ähnliche. Diese können mit weiteren Reagenzien gemischt werden, um die spezifischen Eigenschaften der Beads zu erhalten. Die Herstellung solcher unbeladenen Beads ist z.B. in US-A-4,918,004 Spalte 11, Zeilen 35 bis 59 und US-A-4,828,984 Spalte 8, Zeilen 25 bis 44, sowie in "Flow Cytometry and Sorting", second edition, in dem Artikel von Lea *et al*. auf Seite 369, rechte Spalte, zweiter Absatz beschrieben. Das erfindungsgemäße Verfahren ist jedoch nicht auf Beads der dort beschriebenen Herstellungsweisen beschränkt, sondern es können Beads jedes bioinerten Materials verwendet werden, die ansonsten der hier vorliegenden Beschreibung entsprechen.

Auf der Oberfläche der Beads stehen chemische Gruppen für eine effiziente Kopplung von Proteinen zur Verfügung, bevorzugt sind dies Hydroxyl-, Amino-, Carboxy-, Epoxy- oder Mercaptogruppen. Die Proteine werden nach einer Aktivierung der Beads mittels beispielsweise Bromcyan oder Sulfonylchlorid an diese Gruppen gekoppelt. Auch eine Kopplung über einen oder mehrere Linker oder Quervernetzer ist möglich, wie z.B. die Verwendung von Carbodiimiden, Glutardialdehyd oder N-Succinimidyl-3-(2-pyridyldithio)propionat (SPDP). Auch Biotin, Avidin, Streptavidin und ähnliche Linker können für die Kopplung verwendet werden. Bevorzugt werden die Proteine kovalent an die Beads gebunden, indem zunächst eine auf der Oberfläche befindliche Carboxylgruppe mit Hilfe eines wasserlöslichen Carbodiimids aktiviert wird, z.B. [1-Ethyl-3-(3-dimethylaminopropyl)carbodiimid-HCl] in einem 0,05 M HEPES-Puffer bei pH 4,5 (HEPES = 2-[4-(Hydroxyethyl)-1-Piperazinyl]-Ethansulfonsäure). Die anschließende Beladung mit dem gewünschten Protein oder Proteingemisch erfolgt nach einem Waschschritt durch Suspension der Beads in 0,05 M HEPES-Puffer, worin die Proteine in der gewünschten Menge gelöst sind (z.B. 0,01 - 1 mg/ml, möglich jedoch bis zur Lösungs-Sättigung). Die Suspension wird für mindestens 12 Stunden inkubiert und anschließend die Beladungsdichte kontrolliert.

Diese Beladungsmethode stellt ein Beispiel dar. Andere Möglichkeiten der Beladung der Beads sind dem Fachmann bekannt. Ein anderes Vorgehen beim Beladen schließt die entstehenden Beads nicht von der Verwendung in dem erfindungsgemäßen Verfahren aus.

Die Polymeroberfläche ist vorzugsweise glatt, um eine möglichst gleichmäßige Beladung der darauf gebundenen Antikörper zu bewerkstelligen. Poröse Oberflächen bieten die unerwünschte Möglichkeit, daß auch in den Poren Antikörper haften bleiben, die dann nur erschwert für die Bindung des Signals zur Verfügung stehen. Die Oberfläche kann geladen oder ungeladen sein, hydrophil oder hydrophob, jedoch ist eine negativ geladene, weitgehend hydrophile Oberfläche zu bevorzugen. Diese Art der Oberfläche ist nicht nur einer Zelloberfläche relativ ähnlich, was die Fremdkörperwirkung verringert, sondern verhindert auch eine unspezifische Bindung der Beads an eine Zelle, da diese ebenfalls üblicherweise eine negativ geladenen hydrophile Oberfläche aufweist. Der einfachste Weg um eine solche Oberfläche zu erhalten ist das Ankoppeln von hydrophilen Komponenten wie z.B. Zuckern oder unreaktiven Proteinen. Auch diese kommen auf natürlichen Zelloberflächen vor und wirken nicht als Fremdkörper.

Die Beads werden bevorzugt mit Proteinen, besonders bevorzugt mit Antikörpern beladen, die für das jeweils zu erfassende Signal spezifisch sind. Die Antikörper können monoklonal oder polyklonal sein, und die Beladung mit Antikörpern schließt eine gleichzeitige Beladung mit einer anderen Substanz nicht aus, insbesondere nicht die gleichzeitige Beladung mit einem Agens, das die Immunzellen zur Cytokin-Abgabe anregt. Jede Art von Antikörper (Ig-D-Typ, Ig-M-Typ oder Ig-G-Typ) kann für die Beladung verwendet werden, jedoch werden Antikörper vom Ig-G-Typ bevorzugt.

Die Beladungsdichte der Antikörper auf den Kügelchen ist in jedem Falle so, daß sie nicht limitierend für die Quantität des erfaßten Signals wirkt. Bevorzugt wird die Dichte in einem Bereich von 10³ bis 10⁶ Antikörper pro Bead eingestellt. Die Verteilung der Proteine auf der Oberfläche ist vollkommen gleichmäßig und wird nicht durch ebenfalls auf der Oberfläche gekoppelte andere Moleküle beeinträchtigt.

Für den Nachweis der Bindung des Primärsignals an die gebundenen Antikörper werden freie, also lösliche Antikörper verwendet, an die ein Molekül gekoppelt ist, mit dessen Hilfe ein quantitativ meßbares Signal erhalten werden kann. Die freien Antikörper können ebenfalls monoklonal oder polyklonal sein und von jeden beliebigen Immunglobulin-Typ. Diese Antikörper können entweder ebenfalls spezifisch für das zu detektierende Signalmolekül oder aber spezifisch für den primär entstehenden Antigen-Antikörper-Komplex sein. Auch ein Gemisch dieser beiden Varianten kann Anwendung finden. Das an den löslichen Antikörper gekoppelte Molekül, mit dessen Hilfe ein meßbares Signal erzeugt wird, kann ein niedermolekulares Molekül sein, das von selbst oder nach Anregung Strahlung eines bestimmten Wellenlängenbereichs abgibt oder absorbiert, oder ein Enzym, das eine Reaktion katalysiert, die zu einem meßbaren Signal führt. Beispiele für solche niedermolekularen Moleküle können Farbstoffe sein, die im sichtbaren Wellenlängenbereich absorbieren, unter UV-Licht oder nach Anregung fluoreszieren oder phosphoreszieren, aber auch Moleküle, die radioaktive Strahlung abgeben. Häufige Anwendung finden für solche Untersuchungen Fluorescein, Rhodamine, Hydrochinone, AMCA (Fluorochrom), Quantum Red, Cy3, Cy5 (Fluorochrom der Firma Coulter), Texas Red, PerCP (Fluorochrom der Firma Becton Dickinson), R-Phycoerythrin (PE), C-Phycocyanin, Allophycocyanin (APC). Als gekoppeltes Molekül kommt außerdem jedes Enzym in Frage, das eine Reaktion katalysiert, die zu einem quantitativ meßbaren Signal führt. Hier sind vor allem die alkalische Phosphatase, Peroxidase, β-Galaktosidase und ähnliche Farbstoff entwickelnde Enzyme gebräuchlich. Bevorzugt findet in dem erfindungsgemäßen Verfahren ein monoklonaler Ig-G-Antikörper Anwendung, der spezifisch für das zu detektierende Cytokin ist und an den Fluorescein gekoppelt ist.

Bei dem erfindungsgemäßen Verfahren wird zur Ermittlung der nach einer Stimulation von den Immunzellen abgegebenen Primärsignale eine homogene Mischung von Immunzellen mit den mit Antikörpern beladenen Polymerkügelchen (Beads) hergestellt und in Form einer Suspensionskultur bei vorzugsweise 37 °C und einem pH-Wert im physiologischen Bereich inkubiert. Dabei verteilen sich die Zellen und die Beads gleichmäßig in Form eines Monolayers auf dem Boden des Kulturgefäßes. Bevorzugt werden nach unten gewölbte Kulturgefäße genommen, um ein hohe Zell-Bead-Dichte zu gewährleisten. Aber auch adhärente Zellkulturen, rührende Suspensionskulturen oder jede andere Art von Kulturhaltung ist für die Anwendung des erfindungsgemäßen Verfahrens möglich.

Als Kulturmedien können alle gängigen Medien für Säugetierzellen Verwendung finden, wie zum Beispiel RPMI, DMEM, William's Medium, MEM-Alpha, Leibovitz's L-15 Medium oder auch verschiedene Minium Essential Media. Solche Medien sind käuflich zu erwerben und dem Fachmann bekannt.

Die Stimulation der Zellen zur Signalabgabe kann sowohl durch Antigene wie Anti-CD 3, Anti-CD 28 oder Cytokine erfolgen, wie auch durch andere Wirkstoffe, die von der Zelle als schädigend erkannt werden, wie z.B. Ionophore wie Ionomycin, Phytohemagglutinin (PHA), 12-0-Tetra-Dekanoyl-Phorbol-13-Acetat (TPA) und Phorbol-12-Myristat-13-Acetat (PMA) oder auch durch elektrische Spannung. Bevorzugt werden die Zellen jeweils mit einer Kombination von PMA / Ionomycin oder Anti-CD 3 / IL-2 oder Ionomycin / TPA stimuliert. Die Konzentration der stimulierenden Agenzien und die Verhältnisse der beiden Stimulus-Partner hängt jeweils von den zu stimulierenden Zelltypen ab und wird für jeden Typ optimiert. Auch können die Beads direkt mit einem oder mehreren dieser Stimulantien beschichtet sein.

Das Verhältnis der Zellen zu den Beads kann stark variieren und ist für zu untersuchende Zusammensetzungen von Zellpopulationen zu optimieren. Das Verhältnis von einer einzelnen Signal-abgebenden Zelle zu einem einzelnen Bead kann dabei 0,5:1 bis 2:1 betragen, ist jedoch bevorzugt in einem Mischungsverhältnis von ungefähr 1:1 einzustellen. Beispielsweise ist das optimale Verhältnis bei PBMC's (Zelle:Bead) 1:2, bei polyklonalen T-Zellen (Zelle:Bead) 1:1 bis 2:1. Bei diesem Mischungsverhältnis ist der Kontakt der Zellen mit den Beads besonders gut gewährleistet, und das Erfassen des abgegebenen Primärsignals erfolgt in nächster Nähe. Die Zellen werden in Kultur durch einen Reiz dazu angeregt, ein Primärsignal abzugeben, das durch die an den Beads gebundenen Antikörper abgefangen wird, weitestgehend vollständig und weitestgehend bevor es durch Wiederaufnahme oder Abbau degradiert wird. "Weitestgehend" bedeutet hier, daß die Reizstärke im Versuchsansatz einer derarten Antikörperbeladung der Beads gegenübersteht, daß nicht aufgrund von Sättigung des Signalempfängers ein Anteil des Signals verloren geht. "Weitestgehend" heißt auch, daß die Beads den Zellen so nahe kommen, ohne als Fremdkörper erkannt zu werden, daß sie das abgegebene Signal in der Stärke empfangen können, wie es für die natürliche Zielzelle bestimmt gewesen wäre. Aufgrund der Größe und der Beladung der Beads wird darüberhinaus das Signal von der entsendenden Zelle auch in der Stärke abgegeben, in der es an eine natürliche Zielzelle abgegeben worden wäre, da das Bead eine solche natürliche Zelle nachzuahmen scheint. Wie oben beschrieben ist gerade der unmittelbare Kontakt für die Kommunikation von Immunzellen nötig, um das Signal in der vollen Stärke von der entsendenden Zelle zu erhalten. Durch das "Einfangen" der Signalmoleküle mit Hilfe der Antikörper sind diese vor einer Degradation geschützt. Die Zellkultur wird zusammen mit den beladenen Beads vorzugsweise bis zu 36 Stunden nach dem Signal-auslösenden Reiz vorzugsweise bei 37 °C inkubiert

Der lösliche Antikörper mit dem gekoppelten Molekül zur Detektion kann, muß aber nicht, ebenfalls von Anfang an in der Zellkultur vorliegen. Der lösliche Antikörper wird dem Ansatz in so hohem Überschuß zugegeben, daß alle Antigen-Anitkörper-Bead-Komplexe vollständig quantitativ erkannt werden. Dies heißt, daß der lösliche Antikörper in mindestens 10- bis 100-fachem Überschuß gegenüber der Anzahl des gebundenen Antikörpers vorliegt.

Bevorzugt wird der lösliche Antikörper erst zu den Beads gegeben, nachdem die Zellen zusammen mit den Beads aus der Zellkultur durch Zentrifugation abgetrennt wurden, anschließend zweimal mit einer Pufferlösung mit einem pH-Wert im physiologischen Bereich, bevorzugt PBS, gewaschen und in einem geeigneten Medium resuspendiert wurden. Auch hier gilt die Zugabe des löslichen Antikörpers im entsprechenden Überschuß. Als Medium bietet sich entweder eines der gängigen Zellkulturmedien, oder eine Pufferlösung an, bevorzugt wird ein Phosphatpuffer verwendet. Der pH-Bereich zur weiteren Inkubation liegt im physiologischen Bereich, also etwa zwischen pH 6,5 und 8, bevorzugt bei pH 7,4 bis 7,6. Der lösliche Antikörper wird vorzugsweise bei 4°C zusammen mit den Beads im Dunkeln gehalten und üblicherweise bis zu 4 Stunden inkubiert. Die bevorzugte Inkubationsdauer beträgt üblicherweise 30 min bis 1 Stunde.

Durch anschließende Waschschritte mit einer geeigneten Pufferlösung wird überschüssiger löslicher Antikörper entfernt, die entstandenen BeadAntikörper-Antigen-Antikörper-Komplexe in einem definierten Volumen eines geeigneten Mediums aufgenommen und anschließend einer quantitativen Meßmethode zugeführt. Die bevorzugte Meßmethode des erfindungsgemäßen Verfahrens ist bei der Verwendung von Fluorescein als gekoppeltem Molekül die Flow Cytometrie, jedoch ist jede andere quantitativ auswertbare Meßmethode ebenfalls denkbar. Die verwendete Meßmethode ist vor allem von der Möglichkeit abhängig, die das an den löslichen Antikörper gekoppelte Molekül bietet.

Als geeignete Pufferlösungen für die durchzuführenden Waschschritte können vor allem Puffer verwendet werden, deren höchste Pufferkapazität im physiologischen Bereich liegt, bevorzugt wird PBS ("Phosphat Buffered Saline") oder andere Phosphatpuffer verwendet, deren Herstellung dem Fachmann bekannt, bzw. in der Fachliteratur nachzulesen sind. Es können auch modifizierte Puffer wie Hank's Puffer, Dulbecco's PBS, Earle's Buffered Sahne (EBSS) oder HEPES-Puffer verwendet werden. Den Puffern können weitere Zusätze beigefügt werden, die stabilisierend (z.B. BSA ("Bovine Serum Albumine")) oder destabilisierend (z.B. Natriumazid) wirken können, je nach gewünschter Anwendung.

Die bevorzugte Ausführungsform des erfindungsgemäßen Verfahrens ist ein Testansatz, bei dem wie folgt verfahren wird:

Zellen des Immunsystems von Menschen oder Säugetieren werden mit Beads des Durchmessers 7 µm , die mit für ein Cytokin spezifischen Antikörpern beladen sind in einem Mischungsverhältnis von 1:2 bis 2:1 in homogene Suspension gebracht, in dieser Suspension durch Stimulation mittels eines Reizes zur Abgabe von Primärsignalen in Form von Cytokinen angeregt und 1 - 36 Stunden bei 37°C in RPMI-1640 Medium mit pH 7,4 inkubiert. Anschließend wird die Suspension zweimal mit einem Phosphatpuffer (PBS) mit pH 7,4 gewaschen und in einem definierten Volumen desselben Phosphatpuffers aufgenommen. Nach 1 Stunde Inkubation bei 4 °C und anschließendem dreimaligem Waschen mit Phosphatpuffer pH 7,4, werden lösliche Antikörper in deuthichem Überschuß zum gebundenen Antikörper gegeben, die ebenfalls gegen das zu detektierende Cytokin gerichtet sind. An diese löslichen Antikörper ist Fluorescein gekoppelt. Nach 1 Stunde Inkubation bei 37°C werden überschüssige lösliche Antikörper entfernt und die meßbare Fluoreszenz des Komplexes: Bead-gebundener Antikörper-Cytokin-Antikörper im Flow Cytometer quantitativ bestimmt.

Die Zellen lassen sich außerdem parallel dazu mittels Antikörpern bei entsprechender Vor- und Nachbehandlung färben.

Mit diesem Ansatz werden "Trapping-Faktoren" von bis zu 30.000 erreicht. Das bedeutet, daß eine 30.000-fach höhere Konzentration an Cytokin als Primärsignal von Zellen erfaßt wird, als sie ohne die Nähe der Zellen und der Beads und ohne das "Einfangen" zu messen wäre. Dieser hohe Verstärkungsfaktor bei Verwendung von Beads der erfindungsgemäßen Größe war nicht vorherzusehen, da mit bisherigen Meßmethoden maximal ein Faktor 20 erreicht werden konnte. Durch das "Einfangen" des Cytokins durch die an die Beads gebundenen Antikörper wird dieses so stabilisiert, daß es nicht wie in anderen Verfahren zu einem so starken Signalverlust kommt. Darüberhinaus erfolgt aufgrund der erfindungsgemäßen Wahl der Beadgröße und -beladung eine Signalstärke, die von Immunzellen nur bei direktem Zell-Zellkontakt abgegeben wird. Somit wird mit diesem Ansatz weitestgehend ein physiologischer Zustand der Immunantwort nachgeahmt, der zu einer maximalen Ausbeute sowohl bei der Abgabe der Primärsignals von der Zelle, als auch bei der Detektion des Signals mittels der Antikörperbeladenen Beads führt. Es war überraschend und nicht vorherzusehen, daß sich mit Beads der erfindungsgemäßen Größe und Beladung der physiologische Zustand von kommunizierenden Zellen des Immunsystems so gut nachahmen lassen würde.

Das hier vorgestellte erfindungsgemäße Verfahren hat außerdem den besonderen Vorteil, daß es leicht durchführbare Arbeitsschritte mit standardisierten Meßmethoden vereint, so daß ein Verfahren entsteht, das in bisher bestehenden Laboratorien und Arbeitsanordnungen durchführbar ist, dabei allerdings in einem in-vitro-Ansatz physiologische Gegebenheiten nachahmt und somit bisher nur rudimentär zugängliche Parameter der Zellkommunikation, nämlich die Abgabe von Primärsignalen, meßbar macht.

### Beispiele:

### Beispiel 1: Ermittlung des Meßbereichs, Genauigkeit

Um den optimalen Meßbereich und die Genauigkeit der Konzentrationsbestimmung mittels der verwendeten Beads im Flow Cytometer zu bestimmen, wird eine Kalibrierungsreihe mit rekombinantem IFN-γ vermessen.

### Ansatz:

Für die Konzentrationsbestimmung werden Lösungen definierter Konzentration von rekombinant hergestelltem IFN-γ in PBS hergestellt. In diese Lösungen werden die Beads in einer Menge von 20.000 Beads/100µl zugegeben. Die Ansätze werden 24 Stunden inkubiert Die Beads werden durch Zentrifugation aus der Lösung zurückgewonnen, zweimal mit je 200 µl PBS oder Hank's Puffer, 0,1 % NaN₃, 0,1 % BSA gewaschen und anschließend mit dem mit Fluorescein markierten 2. Antikörper versetzt (10 µg/ml). Nach 30 min Inkubation bei 4°C im Dunkeln werden die Beads nochmals dreimal mit dem oben beschriebenen Puffer gewaschen, in einem geeigneten Volumen dieses Puffers aufgenommen (z.B. 500 µl) und anschließend im Flow Cytometer ausgemessen.
Die abzüglich eines internen Korrekturfaktors bestimmten Konzentrationen sind der folgenden Tabelle 2 zu entnehmen

**Tabelle 2**

| eingesetzte Konz. rekomb. IFN-γ (ng/ml) | ermittelte Konz. an IFN-γ abzüglich Korrektur (ng/ml) |
|---|---|
| 1,25 | 0,73 |
| 5 | 5,04 |
| 10 | 9,03 |
| 100 | 125,1 |

Tabelle 2 zeigt die Genauigkeit der Konzentrationsbestimmung des IFN-γ im Meßbereich zwischen 1 und 100 ng/ml.

### Beispiel 2: Bestimmung der IFN-γ Abgabe von stimulierten T-Zellen:

20.000 T-Zellen werden zusammen mit 20.000 Beads der Größe 7 µm, beladen mit Anti-IFN-γ-Antikörper, in ein Kulturgefäß (96er Mikrotiterplatte) gegeben und darin 24 Stunden bei 37 °C mit der Kombination PMA / Ionomycin in den Konzentrationen PMA 1-10 ng/ml, Ionomycin 0,5-1 µM stimuliert. Anschließend werden die Beads aus der Kultur durch 2 min Zentrifugation bei 1000 Umdrehungen pro Minute (upm) isoliert und zweimal mit je 200 µl Waschpuffer gewaschen, d.h. jeweils in dem angegebenen Volumen suspendiert und erneut mit 1000 upm 2 min zentrifugiert. Die Beads werden in 200 µl des Waschpuffers aufgenommen, und mit 10 µl einer 10 µg/ml Lösung des mit Fluorescein markierten 2. Antikörpers versetzt. Nach 30 min Inkubation bei 4°C im Dunkeln werden die Beads durch Zentrifugation wie oben isoliert, dreimal mit Waschpuffer gewaschen und anschließend im Flow Cytometer vermessen. Der verwendete Waschpuffer ist PBS oder Hank's Puffer unter Zusatz von 0,1% NaN₃ und 0,1% BSA.
Das in Form von IFN-γ abgegebene Signal durch die stimulierten T-Zellen wurde mit dieser Methode bestimmt. Die Konzentration von IFN-γ betrug in diesem Ansatz 83,34 ng/ml.

### Beispiel 3: Trapping Assay, optimales Verhältnis von Beads zu Zellzahl bei polyklonalen T-Zellen:

### Ansatz:

Polyklonale T-Zellen werden zusammen mit Beads der Größe 7 µm, beladen mit Anti-IFN-γ-Antikörpern, 24 Stunden mit OKT 3 (= Anti CD 3 der Firma DAKO) und rekombinantem IL-2 in den Konzentrationen OKT 3 4-5 µg/ml und IL-2 40 U/ml stimuliert. Die Anzahl der Beads wird konstant bei 100.000 gehalten, die Zellzahl variiert. Die Konzentration an abgegebenem IFN-γ wird wie in Bsp. 2 ermittelt. Tabelle 3 gibt die IFN-γ-Konzentrationen wieder.

**Tabelle 3**

| Zellzahl polyklonale T-Zellen | IFN-γ-Konz [ng/ml] |
|---|---|
| 25.000 | 1,59 |
| 50.000 | 3,01 |
| 100.000 | 6,03 |
| 200.000 | 9,34 |

Wie in Tabelle 3 zu sehen, ist die Konzentrationsbestimmung in einem Verhältnisbereich der Zellen:Beads von 0,25:1 bis 2:1 linear, d.h. nur abhängig von der Menge der Signal-produzierenden Zellen.

### Beispiel 4: Kinetik der Abgabe von IFN-γ durch polyklonale T-Zellen:

A. Jeweils 200.000 polyklonale T-Zellen werden mit je 100.000 Beads der Größe 7 µm, beladen mit Anti-IFN-γ-Antikörper, unter Stimulation von OKT 3 und IL-2 in den Konzentrationen OKT 3 4-5 µg/ml und IL-2 40 U/ml jeweils einmal 2, 3, 4 bzw. 5 Stunden inkubiert, und die IFN-γ Abgabe wie oben beschrieben bestimmt. Parallel dazu wird IFN-γ intrazellulär bestimmt. Die intrazelluläre Bestimmung erfolgt in einem Parallelansatz ohne Beads: zunächst erfolgt die Stimulation mit Monensin in der Konzentration 1-3 µM. Die Zellen werden anschließend gewaschen und dann mit 4% Paraformaldehyd fixiert, durch 0,1% Saponin permeabilisiert und mit dem gleichen zweiten fluoreszenzmarkierten Antikörper gefärbt. Die Färbung geht auch im selben Ansatz, wobei 1 Sunde vor Ablauf der Stimulationsdauer Monensin zugegeben wird. Monensin verhindert die Freisetzung des gebildeten Cytokins. Die anschließende Fixierung und Permeabilisierung kann ebenfalls im selben Ansatz erfolgen. Der Anteil der Zellen, in denen intrazellulär das Cytokin nachgewiesen wird, wird als Prozentanteil der Geasamtzellen angegeben.

Tabelle 4 zeigt einerseits den Prozentanteil an Zellen mit intrazellulär gespeichertem IFN-γ, andererseits die abgegebenen IFN-γ-Konzentrationen, die durch "Trapping", d.h. mit Hilfe der Beads ermittelt wurden, bzw. die mit Hilfe eines standartisierten ELISA-Tests (von Pharmingen, gemäß mitgeliefertem Standardprotokoll) ermittelte IFN-γ-Konzentration im Überstand derselben Zellkultur.

**Tabelle 4**

| Stimulationsdauer | intrazell. IFN-γ [%] | Trapping, Konz [pg/ml] | Überst. im ELISA Konz [pg/ml] |
|---|---|---|---|
| 2 Stunden | 2,25 | 580 | 618 |
| 3 Stunden | 2,25 | 540 | 282 |
| 4 Stunden | 3,8 | 560 | 45 |
| 5 Stunden | 2,44 | 1170 | 0 |

B. Jeweils 200.000 polyklonale T-Zellen werden mit je 100.000 Beads der Größe 7 µm, beladen mit Anti-IFN-γ-Antikörper, unter Stimulation von Anti-CD 3 (OKT 3) und IL-2 jeweils einmal 2, 3, 4 bzw. 5 Stunden inkubiert. OKT 3 und IL-2 wurden in den Konzentrationen 4-5 µg/ml und bzw. 40 U/ml eingesetzt. Auch hier wird parallel dazu IFN-γ wie oben beschrieben intrazellulär gemessen.

**Tabelle 5**

| Stimulationsdauer | intrazell. IFN-γ [%] | Trapping, Konz [ng/ml) | Überst. im ELISA Konz [pg/ml] |
|---|---|---|---|
| 2 Stunden | 2,25 | 14,63 | 618 |
| 3 Stunden | 2.25 | 14,02 | 282 |
| 4 Stunden | 3,88 | 16,65 | 45 |
| 5 Stunden | 2.44 | 30,48 | 0 |

Die Tabellen 4 und 5 zeigen, daß das Signal im Trapping-Ansatz über mehrere Stunden quantitativ erfaßt wird, während es im ELISA-Ansatz bei längerer Inkubationszeit verloren geht. Der Trapping-Ansatz fängt das abgegebene Signal auf und hält es stabil, während der ELISA nur die Menge an Signalmolekülen erfaßt, die nach der Zell-Zell-Wechselwirkung "übrig bleibt".

### Beispiel 5: Kinetik der Abgabe von IFN-γ durch stimulierte PBMC's

50.000 PBMC'S werden mit 100.000 Beads der Größe 7 µm, beladen mit Anti-IFN-γ-Antikörpern, zusammengegeben und durch 10 ng/ml TPA und 0,5-1 µM Ionomycin einmal 2 Stunden, einmal 3 Stunden und einmal 4 Stunden in Kultur bei 37°C stimuliert. Die Bestimmung des abgegebenen IFN-γ wird wie in Beispiel 4 beschrieben durchgeführt. Daneben wird wie oben beschrieben der Prozentanteil an intrazellulär IFN-γ positiven Zellen ermittelt.

**Tabelle 6**

| Stimulationsdauer | intrazell. IFN-γ %-Zellanteil | Trapping, Konz.[pg/ml] | Überst. im ELISA Konz. [pg/ml] |
|---|---|---|---|
| 2 Stunden | 24,19 | 3080 | 1764 |
| 3 Stunden | 11,26 | 2090 | 282 |
| 4 Stunden | 11,56 | 4090 | 0 |

### Beispiel 6: Nachweis des "Einfangens" des IFN-γ durch die in der Kultur befindlichen Beads

Um zu zeigen, daß das von den Zellen abgegebene Signal (IFN-γ) von den Beads eingefangen wird, werden Zellen mit und ohne Beads in Kultur stimuliert, und anschließend die IFN-γ-Konzentration im Überstand gemessen.
A. Messung nach Stimulation von T-Zellen:
   Es werden jeweils 200.000 Zellen einmal mit 100.000 Beads, einmal ohne Beads in Kultur mit 4-5 µg/ml Anti-CD 3 und 40 U/ml IL-2 stimuliert, und anschließend mit Hilfe des ELISA-Tests (Pharmingen, Standardprotokoll) die IFN-γ-Konzentration im Überstand bestimmt.
B. Messung nach Stimulation von PBMC's:
   Jeweils 50.000 PBMC's werden einmal mit 100.000 Beads, einmal ohne Beads in Kultur durch 0,5-1 µM Ionomycin und 1-10 ng/ml PMA stimuliert, und wie unter A. die Konzentration von IFN-γ im Überstand bestimmt.

Tabelle 7 zeigt die ermittelte IFN-γ-Konzentration im Überstand der stimulierten Zellen. Es ist deutlich zu sehen, daß die Beads das IFN-γ in allen Ansätzen fast vollständig "eingefangen" haben, da praktisch kein IFN-γ mehr im Überstand zu messen ist.

**Tabelle 7**

| Stimulationsdauer | T-Zellen mit Beads | T-Zellen ohne Beads | PBMC's mit Beads | PBMC's ohne Beads |
|---|---|---|---|---|
| 2 Stunden | 45 | 618 | 45 | 1764 |
| 3 Stunden | 0 | 282 | 0 | 282 |
| 4 Stunden | 0 | 45 | 0 | 0 |
| 5 Stunden | 45 | 0 | n.g. | n.g. |
| Abkürzungen: PBMC's = Peripheral Blood Mononuclear Cells, n.g. = nicht gemessen | | | | |

### Zusammenfassung der Vorteile des Bead-Trapping-Assays:

- Der Trappingassay mißt die Gesamtmenge des Cytokins, das gebildet wird, der ELISA jeweils nur die Menge an Cytokin, die nach mehrmaligem Signalaustausch übrig bleibt.
- Schon ab 2 Stunden ist die Kinetik von IFN-γ quantitativ auswertbar.
- Es wird nur eine relativ geringe Zellzahl für die Messung des Signals benötigt, nämlich 2 x 10⁴ bis 2 x 10⁵ pro Ansatz.
- Es ist eine physiologische Stimulation der lebenden Immunzellen möglich, z.B. mit Anti-CD 3, IL-2 oder Antigenen.
- Die Zellen können lebend weiter untersucht werden.

## Patentansprüche

1. Verfahren zum Erfassen von Primärsignalen der Zellkommunikation von menschlichen und Säugetierzellen außerhalb eines Organismus, wobei Polymerkügelchen mit einem Durchmesser von 5 bis 100 µm, die mindestens mit einem für diese Primärsignale spezifischen Antikörper beladen sind, mit menschlichen oder Säugetierzellen, die Primärsignale aussenden können, gemischt und in Zellkultur unter physiologischen Bedingungen inkubiert werden, worin die Zellen durch einen externen Reiz zur Abgabe des Primärsignals stimuliert werden und die Polymerkügelchen die abgegebenen Primärsignale auffangen, welche anschließend mit Hilfe eines weiteren Antikörpers, an den ein die Detektion zulassendes Molekül gekoppelt ist, meßbar gemacht werden.

2. Verfahren nach Anspruch 1, dadurch gekennzeichnet, daß die erfaßten Primärsignale Cytokinabgaben von Immunzellen sind.

3. Verfahren nach Anspruch 2, dadurch gekennzeichnet, daß das abgegebene Cytokin aus Interleukinen (IL), Interferonen (IFN), Tumor-Nekrose-Faktoren (TNF) oder Transforming Growth Factoren (TGF) ausgewählt ist.

4. Verfahren nach Anspruch 3, dadurch gekennzeichnet, daß das abgegebene Cytokin Interferon-γ ist.

5. Verfahren nach einem der Ansprüche 1 bis 4, dadurch gekennzeichnet, daß die verwendeten Polymerkügelchen einen Durchmesser von 5 bis 30 µm haben.

6. Verfahren nach einem der Ansprüche 1 bis 4, dadurch gekennzeichnet, daß die verwendeten Polymerkügelchen einen Durchmesser von 6 bis 10 µm haben.

7. Verfahren nach einem der Ansprüche 1 bis 4, dadurch gekennzeichnet, daß die verwendeten Polymerkügelchen einen Durchmesser von 7 µm haben.

8. Verfahren nach einem der Ansprüche 1 bis 7, dadurch gekennzeichnet, daß die Polymerkügelchen und die signalabgebenden Zellen in einem Mischungsverhältnis von 0,5 : 1 bis 2 : 1 zusammengegeben werden.

9. Verfahren nach einem der Ansprüche 1 bis 7, dadurch gekennzeichnet, daß die Polymerkügelchen mit den signalabgebenden Zellen in einem Mischungsverhältnis von 1 : 1 zusammengegeben werden.

10. Verfahren nach einem der Ansprüche 1 bis 9, dadurch gekennzeichnet, daß die Polymerkügelchen mit den Zellen in direkten Kontakt treten können.

11. Verfahren nach einem der Ansprüche 1 bis 10, dadurch gekennzeichnet, daß der zweite Antikörper spezifisch den Komplex von erstem Antikörper mit dem gebundenen Cytokin erkennt.

12. Verfahren nach einem der Ansprüche 1 bis 10, dadurch gekennzeichnet, daß der zweite Antikörper spezifisch ein Cytokin erkennt.

13. Verfahren nach einem der Ansprüche 1 bis 12, dadurch gekennzeichnet, daß an den zweiten Antikörper ein niedermolekulares, leicht zu detektierendes Farbstoffmolekül gekoppelt ist.

14. Verfahren nach Anspruch 13, dadurch gekennzeichnet, daß das Farbstoffmolekül Fluorescein ist.

15. Verfahren nach einem der Ansprüche 1 bis 17, dadurch gekennzeichnet, daß verwendete Medien und Pufferlösungen einen pH zwischen 6,5 und 8 haben.
